# EUROPEAN PATENT SPECIFICATION

(11) **EP 2 129 324 B1**
(45) Date of publication and mention of the grant of the patent: **11.02.2015**
(21) Application number: 08725862.0
(22) Date of filing: 21.02.2008
(51) Int. Cl.: A61F 2/04, A61F 5/00

(54) **IMPLANTABLE WEIGHT CONTROL DEVICE**
IMPLANTIERBARE GEWICHTSKONTROLLVORRICHTUNG
DISPOSITIF IMPLANTABLE DE LIMITATION DU POIDS

(30) Priority: 27.02.2007 US 711364; 27.02.2007 US 711363
(43) Date of publication of application: 09.12.2009
(73) Proprietor: Agt, Inc., Rancho Mirage, CA 92270 (US)
(72) Inventor: ANNUNZIATA, Gary, Rancho Mirage, CA 92270 (US)
(74) Representative: Müller-Boré & Partner Patentanwälte PartG mbB
(86) International application number: PCT/US2008/002269
(87) International publication number: WO 2008/106041

(56) References cited:
- WO-A2-2006/020370
- US-A- 5 084 061
- US-A- 5 330 486
- US-A1- 2005 273 060
- US-A1- 2005 273 060

## Description

### TECHNICAL FIELD

The present invention provides an inflatable weight control device that is implantable with an endoscope. Once inflated, the device is retained within the patient's pyloric valve to form a gastric outlet obstruction wherein chyme can only pass through a central passageway in the device to reach the patient's duodenum.

### BACKGROUND OF THE INVENTION

According to the Center for Disease Control (CDC), the prevalence of overweight and obesity has increased sharply for both adults and children over the past 30 years. Between 1976-1980 and 2003-2004, the prevalence of obesity among adults aged 20-74 years increased from 15.0% to 32.9%. Among young people, the prevalence of overweight increased from 5.0% to 13.9% for those aged 2-5 years, 6.5% to 18.8% for those aged 6-11 years, and 5.0% to 17.4% for those aged 12-19 years. Overweight and obesity ranges are determined by using weight and height to calculate a number called the "body mass index" (BMI). BMI is used because, for most people, it correlates with their amount of body fat. An adult who has a BMI between 25 and 29.9 is considered overweight, while an adult who has a BMI of 30 or higher is considered obese. Within the obesity category, a person is morbidly obese if he meets one of three criteria: a BMI over 35, at least 100 lbs. overweight, or 100% above ideal body weight; and a person is super-obese if he weighs in excess of 350 lbs.

It is well recognized that being overweight or obese raises many significant health implications. For example, obesity increases the risk of many diseases and health conditions, including: hypertension, dyslipidemia (for example, high total cholesterol or high levels of triglycerides), type 2 diabetes, coronary heart disease, stroke, gallbladder disease, osteoarthritis, sleep apnea, and respiratory problems. In addition to the health implications, overweight and obesity have a significant economic impact on the U.S. health care system. Medical costs associated with overweight and obesity may involve direct and indirect costs. Direct medical costs may include preventive, diagnostic, and treatment services related to obesity. Indirect costs relate to morbidity and mortality costs, where morbidity costs are defined as the value of income lost from decreased productivity, restricted activity, absenteeism, and bed days, and mortality costs are the value of future income lost by premature death. According to a study of national costs attributed to both overweight (BMI 25-29.9) and obesity (BMI greater than 30), medical expenses accounted for 9.1 percent of total U.S. medical expenditures in 1998 and may have reached as high as $78.5 billion ($92.6 billion in 2002 dollars). Approximately half of these costs were paid by Medicaid and Medicare. A more recent study focused on state-level estimates of the total obesity attributable direct medical expenditures. State-level estimates range from $87 million (Wyoming) to $7.7 billion (California). Obesity-attributable Medicare estimates range from $15 million (Wyoming) to $1.7 billion (California), and obesity-attributable Medicaid expenditures range from $23 million (Wyoming) to $3.5 billion (New York). The state differences in obesity-attributable expenditures are partly driven by the differences in the size of each state's population.

According to the CDC, overweight and obesity are a result of energy imbalance over a long period of time due to a combination of several factors. These factors include, individual behaviors, environmental factors, and genetics. Energy imbalance results when the number of calories consumed is not equal to the number of calories used. When the quantity of calories consumed is greater than calories used, weight gain results. In the United States and many other highly developed countries, the growing prevalence of pre-packaged foods, fast food restaurants, and soft drinks, that tend to be high in fat, sugar, and calories, increase a person's calorie consumption. In addition, portion size has also increased which causes people to eat more during a meal or snack, thereby increasing their calorie consumption. If the body does not burn off the extra calories consumed from larger portions, fast food, or soft drinks, weight gain will likely occur. Despite the well-known benefits of being physically active, most Americans lead a sedentary life style. According to the Behavioral Risk Factor Surveillance System, in 2000 more than 26% of adults reported limited or no physical activity during the course of an average week. Regarding the environmental factor, people may make decisions based on their environment or community. For example, a person may choose not to walk to the store or to work because of a lack of sidewalks. Genetics have been proven to play a role in obesity. For example, genes can directly cause obesity in disorders such as Bardet-Biedl syndrome and Prader-Willi syndrome. However, genes do not always predict future health; in some cases multiple genes may increase one's susceptibility for obesity and require outside factors, such as abundant food supply or little physical activity.

Conventional approaches to combat overweight and obesity have led doctors to surgically modify patients' anatomies in an attempt to reduce consumption by inducing satiety or a "full" feeling in the patient, thereby reducing the desire to eat. Examples include stomach stapling, or gastroplasties, to reduce the volumetric size of the stomach. In addition, two procedures, the Roux-en-Y gastric bypass and the biliopancreatic diversion with duodenal switch (BPD), reduce the size of the stomach and the effective-length of intestine available for nutrient absorption. These two procedures reduce the stomach volume and the ability of a patient to consume food. In an attempt to limit nutrient absorption in the digestive tract, at least one company has introduced a sleeve that is implanted in obese patients. U.S. Patent No. 7,025,791 discloses a bariatric sleeve that is anchored in the stomach and extends through the pylorus and duodenum and beyond the ligament of Treitz. All chyme exiting the stomach is funneled through the sleeve and bypasses the duodenum and proximal jejunum. By directing the chyme through the sleeve, the digestion and absorption process in the duodenum is interrupted because the chyme cannot mix with the fluids in the duodenum. Because there is no mixing of bile with the chyme until the jejunum, the absorption of fats and carbohydrates is reduced. Although these conventional methods and approaches have had some success, they suffer from a number of limitations including high correction and mortality rates. Also, conventional methods are costly and prone to adaptation by the patient's digestive tract which reduces the effectiveness of the method.

Further, WO 2006/020370 A2 discloses a device for performing one or more functions in a gastrointestinal tract of a patient including an anchoring member and at least one actuator, sensor, or combination of both coupled with the anchoring device. The anchoring device is adapted to maintain at least part of the device within a pyloric portion of the patient's stomach and to intermittently engage, without directly attaching to, stomach tissue. The preamble of appended claim 1 is based on this disclosure.

US 5,084,061 A discloses an intragastric balloon having an ellipsoid configuration so that the balloon implanted in the stomach tends to rotate or rock only about one axis when a surgeon attempts to manipulate the balloon, for example, for the purpose of finding a filler valve and inserting a filler tube into it. The filler valve is disposed on the equator. A retrieval tab is mounted to the exterior of the balloon, to permit capturing of the balloon and retrieval from the stomach, after the balloon has been deflated and is no longer desired for weight control purposes.

Further, US 2005/0273060 A1 discloses an apparatus for treating obesity including an artificial fistula created between gastrointestinal organs such as between the stomach and the colon. The implant comprises a passageway having an internal lumen with an inlet end and an outlet end.

Accordingly there is a need for an implantable weight loss device that is effective in prompting satiety while being minimally invasive and not irritable to patients over time. At the same time, there is a need to provide a weight control device that can be implanted with an endoscope during a visit to a doctor's office, and that does not require a hospital visit. Finally, it would be advantageous to provide treatment methods for combating overweight or obesity based upon the weight loss device that forms a gastric outlet obstruction in the stomach to prompt satiety and reduce food consumption.

### SUMMARY OF THE INVENTION

The present invention provides a weight control device as defined in claim 1. The weight control device resides within the pylorus and between the duodenum and stomach. The weight control device includes an internal passageway which forms a conduit for the reception and passage of chyme from the stomach through the pylorus and to the duodenum. In particular, the above needs are supplied by the weight control device according to claim 1.

According to one aspect of the invention, the weight control device includes a first bulb, a second bulb, and an intermediate portion which collectively define an inflatable body. The internal passageway extends through the body, wherein the passageway receives and allows for the passage of chyme from the stomach to the duodenum. In a use position, the first bulb engages an inner surface of the pyloric antrum. This engagement prevents chyme from passing there between and as a result, chyme must pass through the internal passageway to exit the stomach. In the use position, the second bulb engages an inner surface of the pyloric canal, wherein the second bulb resides between the duodenum and the pyloric valve. Also in the use position, the intermediate portion of the body engages an inner surface of the pyloric valve.

According to another aspect of the invention, the collapsed body is inserted through the patient's mouth and through both the esophagus and stomach with the endoscope. A filling tube associated with the endoscope supplies saline through the valve and into the body until the device is sufficiently inflated to form the gastric outlet obstruction. To remove an implanted device, the body is deflated, such as by piercing the first bulb, and the endoscope is used to remove the deflated body.

As an example, which does not form part of the present invention, methods of treating overweight and/or obesity may involve the inventive device. A first treatment method involves the staggered implantation of devices having different sized internal passageways to counter the digestive tract's accommodation of an implanted device. In a first treatment step, a first device having an internal passageway with a first diameter is implanted within the patient's pylorus. When the digestive tract adapts to the first device and weight loss stagnates, a second treatment step is employed. The second treatment step involves the replacement of the first device with a second device having an internal passageway that is smaller than that of the first device. While the second device continues to provide a gastric outlet obstruction in the stomach that blocks the normal passage of chyme from the stomach and that redirects chyme into the passageway, the passageway has reduced dimensions that reduce the volume of chyme that may pass through the device. When the digestive tract adapts to the second device and weight loss again stagnates, a third treatment step is commenced. The third treatment step consists of replacing the second device with a third device having an internal passageway that is smaller than both the first and second devices. Like the first and second devices, the third device provides a gastric outlet obstruction in the stomach that blocks the normal passage of chyme from the stomach and that redirects chyme into the passageway. Because less chyme is able to pass through the passageway of the third device compared to the passageway of both the first and second devices, an even greater amount of chyme accumulates proximate the first bulb and within the stomach leading the patient to feel full and stop consuming food.

A second treatment method involves the sequenced use of removable inserts placed within the passageway to counter the digest tract's accommodation of the device. In a first stage of the treatment method, the device is implanted within the patient's pylorus. When the patient's digestive tract begins to accommodate the device and weight loss stagnates, the second stage of the treatment method commences by placing a first insert into the passageway to reduce the diameter of the passageway. Depending upon whether the digestive track continues to adapt to the device, different sized inserts may be employed to reduce the volumetric capacity of the passageway. As a result, the amount of chyme that may pass through the device is reduced, which increases the accumulation of chyme within the stomach, leading the patient to feel full and stop consuming food.

Other features and advantages of the invention will be apparent from the following specification taken in conjunction with the following drawings.

### BRIEF DESCRIPTION OF THE DRAWINGS

To understand the present invention, it will now be described by way of example, with reference to the accompanying drawings in which:
FIG. 1 is a perspective view of a weight control device implanted within a patient's stomach, wherein the weight control device provides a valve assembly 40, which does not form part of the present invention;
FIG. 2 is a perspective view of the weight control device of Fig. 1;
FIG. 3 is a cross-sectional view of the weight control device of Fig. 1, showing an internal passageway of the device;
FIG. 4 is a perspective view of the weight control device of Fig. 1, showing the device in a pre-installed state wherein the device is deflated;
FIG. 5 is a perspective view of the weight control device of Fig. 1, showing the device during one step of the implantation process;
FIG. 6 is a perspective view of the weight control device of Fig. 1, showing the device during a second step of the implantation process;
FIG. 7 is a perspective view of a weight control device, according to the present invention, having the internal valve assembly 65;
FIG. 8 is a cross-sectional view of the weight control device of Fig. 1, showing a removable insert positioned within the passageway of the device;
FIG. 9 is a partial cross-sectional view of another embodiment of a weight control device, showing a first-sized removable insert positioned within the passageway of the device;
FIG. 10 is a partial cross-sectional view of the weight control device of Fig. 9, showing a second-sized removable insert positioned within the device passageway;
FIG. 11 is a partial cross-sectional view of the weight control device of Fig. 9, showing a third-sized removable insert positioned within the device passageway;
FIG. 12 is a cross-sectional view of another embodiment of a weight control device, showing the device having two internal passageways;

### DETAILED DESCRIPTION

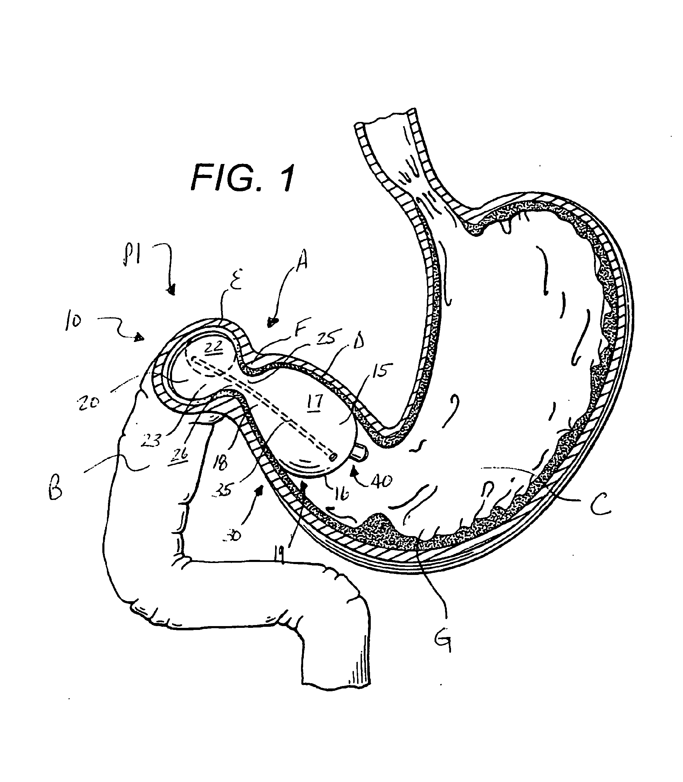

Figs. 1-12 depict an inflatable weight control device 10 that is implanted in a patient's digestive track to form a gastric outlet obstruction 19. As explained in greater detail below, an endoscope is used to implant the weight control device 10 within the pylorus A and between the duodenum B and stomach C. While the human digestive track includes many components, only those that are relevant to the present invention are shown in the Figures. The pylorus A is the region of the stomach C that connects to the duodenum B, and that includes three parts: the pyloric antrum D which connects to the body of the stomach C; the pyloric canal E which connects to the duodenum B; and, the pyloric sphincter or valve F which is a ring of muscle that allows for the passage of chyme from the stomach C to the duodenum B. Once inflated, the configuration of the device 10 retains it within the pylorus A and prevents unintended movement into the duodenum B or stomach C. The stomach C consists of four coats or layers: the serous coat, the muscular coat, the areolar or submucous coat, and the mucous membrane, together with an assortment of vessels and nerves. The weight control device 10 includes an internal passageway which forms a conduit for the reception and passage of chyme from the stomach through the pyloric valve and to the duodenum. Chyme is the liquid substance produced in the stomach C before passing through the pyloric valve F and entering the duodenum B. Chyme is highly acidic (a pH value of approximately 2) and consists of partially digested food, water, hydrochloric acid, and various digestive enzymes. In the absence of the inventive device 10, chyme passes through the pyloric valve F and into the duodenum B, where the extraction of nutrients begins.

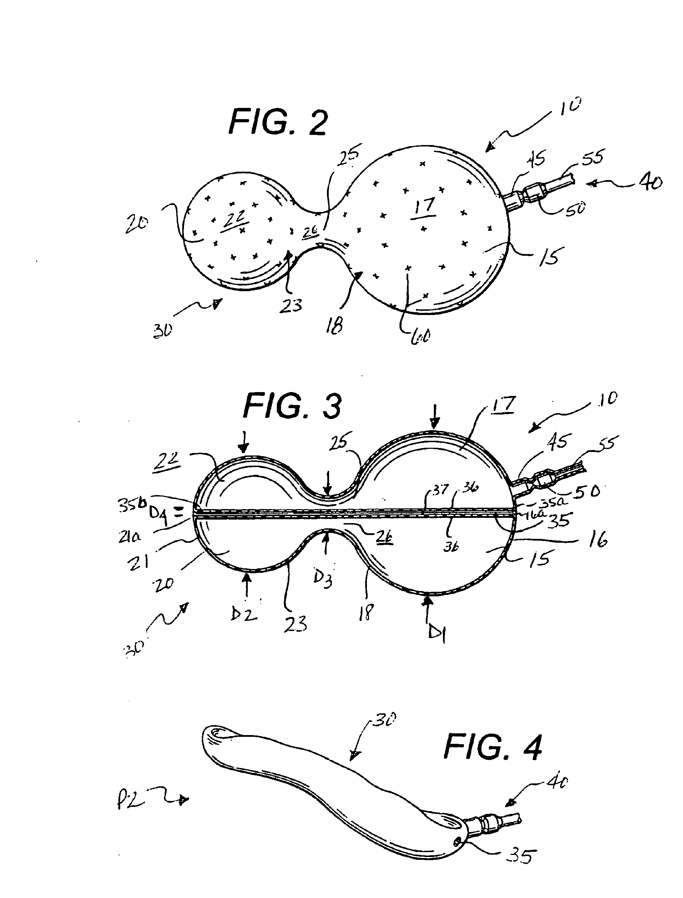

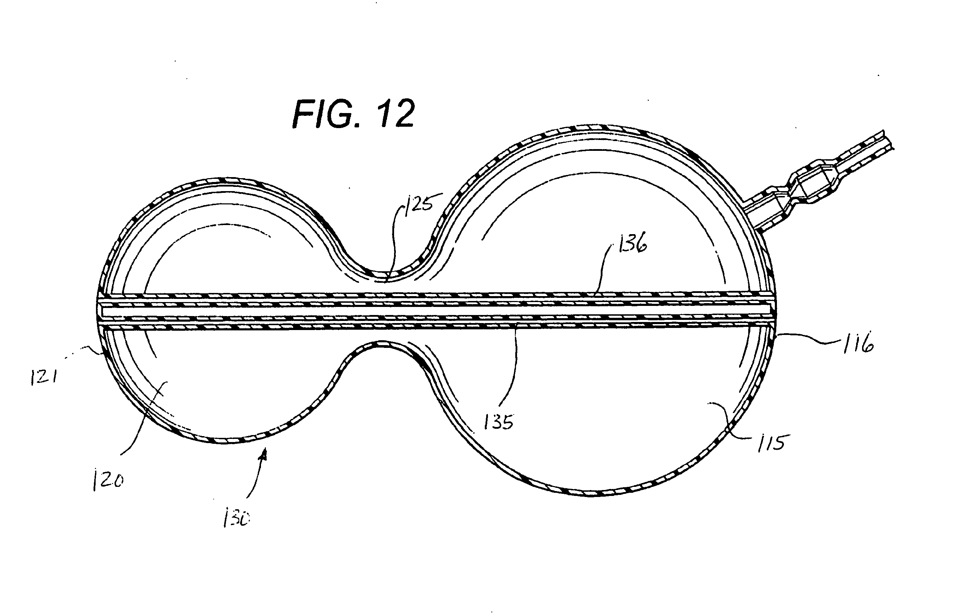

The weight control device 10 includes a first bulbous portion or first bulb 15, a second bulbous portion or second bulb 20, and an intermediate portion 25. These components collectively define an inflatable body 30 with a "dumbbell" configuration. As shown in Fig. 1, the body 30 extends between the pyloric antrum D and the pyloric canal E, and through the pyloric valve F. Unlike conventional weight control devices, such as the elongated sleeve of U.S. Patent No. 7,025,791 that extends through the duodenum B, the second bulb 20 resides within the pyloric canal E and does not extend into the duodenum B. Also unlike the sleeve of the '791 Patent, the body 30 has opposed cavities or bulbs 15, 20 that extend from a common segment, the intermediate portion 25, and that is inflatable with the endoscope H. The intermediate portion 25 is semi-rigid compared to the more flexible first and second bulbs 15, 20, wherein the contractions of pyloric valve F do not collapse the intermediate portion. To increase the rigidity of the intermediate portion 25, the wall thickness of the intermediate portion 25 is greater than that of the first and second bulbs 15, 20. Unlike the sleeve of the '791 Patent, which holds the pyloric valve F open to induce a "dumping syndrome," the intermediate portion 25 is engaged and contracted to a small extent by the pyloric valve F. The device 10 includes at least one internal passageway or lumen 35 extending through the body 30, wherein the passageway 35 receives and allows for the passage of chyme from the stomach C to the duodenum B. Referring to Fig. 3, the internal passageway 35 extends from an end wall 16 of the first bulb 15 through the intermediate portion 25 and to an end wall 21 of the second bulb 20. Further, the passageway 35 includes a first end 35a aligned with an opening 16a in the end wall 16, and a second end 35b aligned with an opening 21a in the end wall 21. In a preferred embodiment of the weight control device 10, the passageway 35 represents the longitudinal axis of the body 30. The internal passageway 35 is defined by two substantially parallel walls 36 that are spaced a distance apart to define a cavity 37. In the embodiment of Fig. 12, the body 130 includes a first internal passageway 135 and a second internal passageway 136 extending between the end wall 116 of the first bulb 115 and the end wall 121 of the second bulb 120, and through the intermediate portion 125. Thus, the first and second passageways 135, 136 provide the body 130 with two conduits for the passage of chyme from the stomach C to the duodenum B. In a slight variation of the body 130, the first and second internal passageways 135, 136 may converge, such as aft of the intermediate portion 125, to define a common exit passageway in the second bulb 120.

When the weight control device 10 is implanted and inflated to define an installed or use position P1 (see Fig. 1), an exterior surface 17 of the first bulb 15 engages an inner surface of the pyloric antrum D. Thus, the first bulb 15 resides between the stomach corpus G (the central body portion of the stomach) and the pyloric valve F. In the use position P1, the first bulb 15 effectively seals the pyloric antrum D to prevent the normal flow of chyme from the stomach B into the pyloric valve F and forces or directs chyme into the internal passageway 35. Because the body 30 is inflatable, the dimensions of the first bulb 15 can be customized during inflation to match the dimensions of the pyloric antrum D to facilitate sealing engagement between the bulb surface 17 and the inner surface of the pyloric antrum D. This sealing engagement results in a gastric outlet obstruction 19 (see Fig. 1) that causes chyme to accumulate proximate the first bulb 15 prior to entering the internal passageway 35. The obstruction also prevents chyme from departing the pyloric antrum D and as a result, chyme must pass through the internal passageway 35 to exit the stomach. As a result of the engagement, the first bulb 15 provides a gastric outlet obstruction 19 in the stomach C that (i) prevents the normal passage of chyme from the stomach C through the pylorus; (ii) redirects chyme into the passageway 35; and, (iii) reduces the volumetric capacity of the stomach C thereby causing the patient to feel satiated or "full" after consuming a reduced amount of food. Lastly, the engagement between the first bulb 15 and the pyloric antrum D retains the body 30 in the use position P1 such that the properly inflated first bulb 15 cannot pass beyond the pyloric valve F and into the duodenum B. As shown in Fig. 1, the first bulb 15 has exterior dimensions that exceed the dimensions of the pyloric valve F whereby the first bulb 15 is prevented from passing beyond the valve F and into the duodenum B. In a preferred embodiment of the device 10, a first interface region or shoulder 18 (see Figs. 1-3) is defined between the first bulb 15 and the intermediate portion 25. The interface region 18 has a tapered, annular configuration and engages the pyloric antrum D adjacent to the pyloric valve F. In the use position P1, the first bulb 15 has a diameter D1 that is 4-9 centimeters (cm), and preferably the diameter D1 is 5-8 cm.

In the use position P1, an exterior surface 22 of the second bulb 20 engages an inner surface of the pyloric canal E, wherein the second bulb 20 resides between the duodenum B and the pyloric valve F. The engagement between the second bulb 20 and the pyloric canal E retains the body 30 in the use position P1 such that the properly inflated second bulb 20 cannot pass through the pyloric valve F and into the pyloric antrum D or stomach corpus G. Referring to Fig. 1, the opening 21a in the end wall 21 of the second bulb 20 is oriented such that chyme discharged from the passageway 35 is directed into the duodenum B. In a preferred embodiment of the device 10, a second interface region or shoulder 23 (see Figs. 1-3) is defined between the second bulb 20 and the intermediate portion 25. The interface region 23 has a tapered, annular configuration and engages the pyloric canal E adjacent to the pyloric valve F. Once inflated and implanted within a patient, the second bulb 20 has a diameter D2 that is 2-5 cm and preferably the diameter D2 is 3-4 cm. Also in the use position P1, an exterior surface 26 of the intermediate portion 25 engages an inner surface of the pyloric valve F, wherein the first bulb 15 engages the pyloric antrum B and the second bulb 20 engages the pyloric canal E. Described in a different manner, the pyloric valve F contracts about the intermediate portion 25. In the embodiment where the intermediate portion 25 is semi-rigid, the contraction of the pyloric valve F does not cause the intermediate portion 25 or the internal passageway 35 to completely collapse. In the use position P1, the intermediate portion 25 has a diameter D3 that is 1.0-2.5 centimeters, and preferably the diameter D3 is 1.5-2.0 cm. Due to the configuration of the body 30, the intermediate diameter D3 is less than both the first bulb diameter D1 and the second bulb diameter D2. The internal passageway 35 has a diameter D4 that is 1-6 millimeters (mm), and preferably the diameter D4 is 2-4 mm.

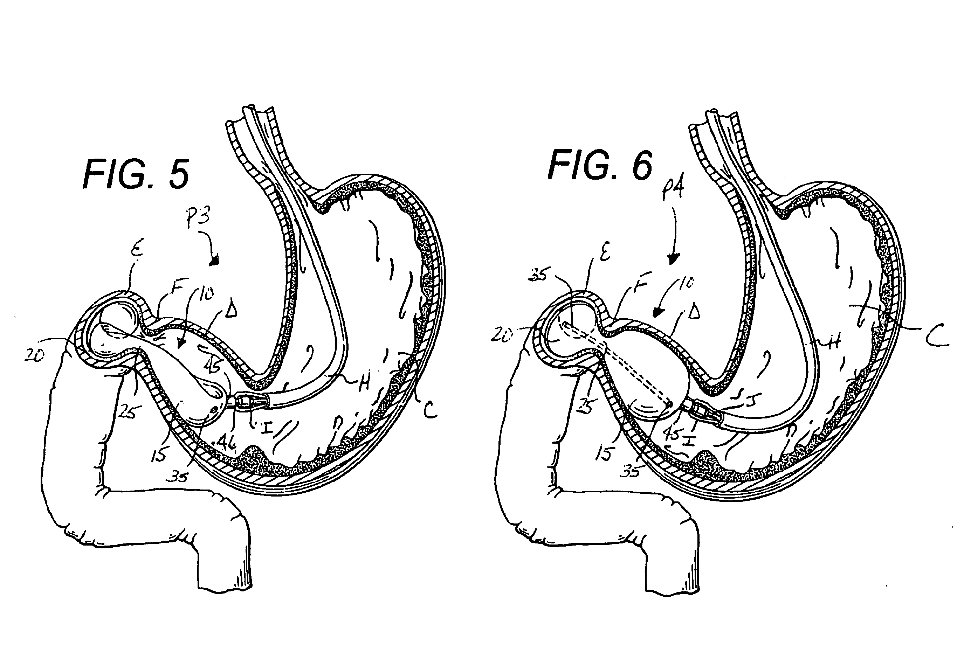

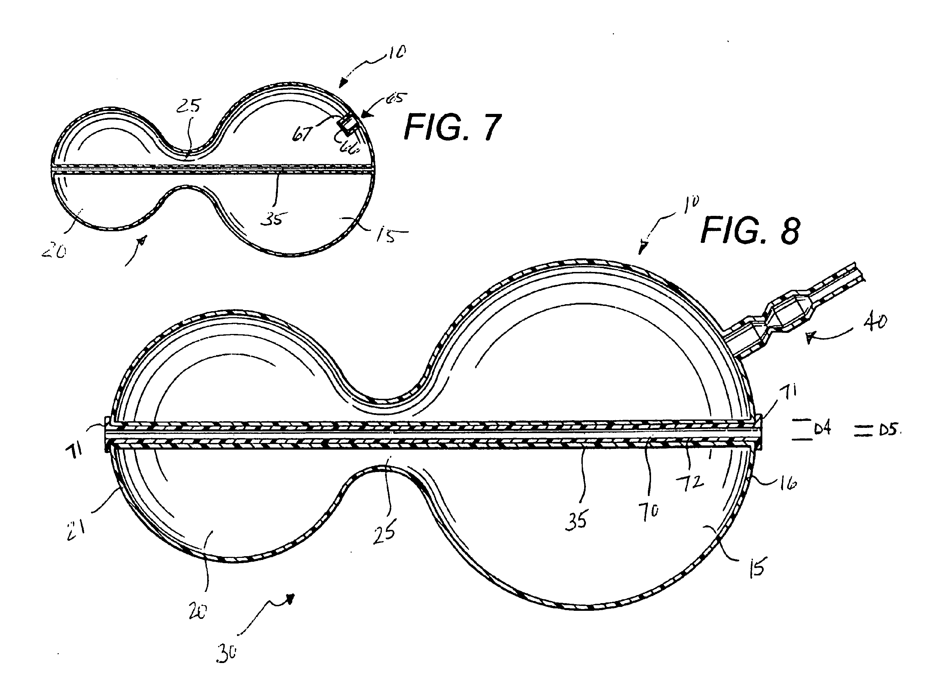

Fig. 4 depicts the device 10 in a collapsed or un-inflated position P2, wherein the device 10 is flexible and ready to be implanted in a patient with the use of an endoscope H. A valve assembly 40 that is used to fill the device 10 with an inflation fluid, such as saline, extends from the first bulb 15. The valve assembly 40 includes a stem 45 that is grasped by the endoscope H (see Figs. 5 and 6) during the implantation process, a valve body 50, and a fill tube segment 55 that can be severed after the device reaches the installed position P1. The valve body 50 has an internal, one-way valve that accepts the inflation fluid supplied by the endoscope H. Over time, the body 30 may require additional inflation fluid due to seepage or leakage. Alternatively, the valve body 50 has a two-way valve that allows for both the filling and removal of inflation fluid from the body 30. The valve assembly 40 extends at off-center location from the first bulb 15 because the internal passageway 35 occupies the central axis of the body 30. In the event that the passageway 35 is not centrally located in the body 30, the valve assembly 40 should be offset from the passageway 35. According to the invention, the body 30 includes an internal valve assembly 65 (see Fig. 7) that extends inward from an outer surface of the first bulb 15. Like the valve assembly 40 of Figs. 1-6, the assembly 65 includes a valve body 66 and an internal stem 67. As shown in Fig. 2, the body 30 includes a plurality of radio opaque markers 60 that facilitate detection of the device 10 during testing subsequent to implantation. The counter the acids in the stomach A and to ensure a sufficient life of the body 30, the device 10 can be fabricated from one of the following materials: fluoropolymer, such as PTFE (polytetrafluoroethylene), PFA (perfluoroalkoxy polymer resin), FEP (fluorinated ethylenepropylene) or polyethylene, such as HDPE (high density polyethylene), MDPE (medium density polyethylene) or LDPE (low density polyethylene).

Fig. 5 depicts an intermediate position P3 where the collapsed device 10 has been inserted through the stomach C and into the pylorus A by the endoscope H. The collapsed device 10 is inserted through the patient's mouth and through both the esophagus and stomach with the endoscope H. The endoscope H has a clamp I that grasps a groove 46 of the stem 45 to facilitate insertion of the device 10. In the intermediate position P3, the first bulb 15 resides within the pyloric antrum D, the second bulb 20 resides within the pyloric canal, and the intermediate portion 25 resides within the pyloric valve F. A filling tube J within the endoscope H supplies an inflation fluid, such as saline, through the valve 50 and into the body 30 until an inflated position P4 (see Fig. 6) is reached. Since the body 30 is an enclosed vessel, the inflation fluid fills the first bulb 15, the second bulb 20 and the intermediate portion 25 to inflate the body 30. In the inflated position P4, the endoscope H is coupled to the inflated body 30, wherein the exterior surface 17 of the first bulb 15 engages an inner surface of the pyloric antrum D, the exterior surface 22 of the second bulb 20 engages an inner surface of the pyloric canal E, and the exterior surface 26 of the intermediate portion 25 engages an inner surface of the pyloric valve F. Thus, in the inflated position P4, the device 10 is inflated such that the first bulb 15 engages the pyloric antrum B and the second bulb 20 engages the pyloric canal E, both adjacent the pyloric valve F. After the inflated position P4 is reached, the endoscope H is de-coupled from the valve assembly 40 and removed from the patient whereby the device 10 is ready for use. To remove an implanted device 10, the body 30 is deflated, such as by piercing the first bulb 15, and the endoscope H is used to remove the deflated body 30.

After the device 10 has been implanted and is in the use position P1, the device 10 may be used in a method of treating obese patients. The method comprises the sequenced implantation of devices 10 having different sized passageways 35 to counter the digestive tract's accommodation of an implanted device 10. In a first treatment step, a first device 10 having the internal passageway 35 with a diameter D4, such as 4.0 millimeters, is implanted within the patient's pylorus A at the use position P1. Thus, the first bulb 15 provides a gastric outlet obstruction 19 in the stomach C that blocks the normal passage of chyme from the stomach C and that redirects chyme into the passageway 35. The resulting gastric outlet obstruction 19 reduces the volumetric capacity of the stomach C thereby causing the patient to feel "full" resulting in appetite suppression, after consuming a reduced quantity of food. Initially, the patient will feel full and stop consuming food or reduce the rate of food consumption, which will lead to an initial phase of weight loss during the first treatment step. However, over time, the patient's digestive tract will adapt to the first device 10, the patient will not feel full after consuming a similar quantity of food, and the weight loss experienced during the initial phase will stagnate.

A second treatment step is designed to combat the stagnation in weight loss experienced during the first step by using a different-sized second device 10 in place of the first device 10. Specifically, the first device 10 is removed from the patient and replaced by the second device 10 which has an internal passageway 35 with a reduced diameter D4, such as 3.0 mm. Thus, the passageway diameter D4 of the second device 10 is less than that of the first device 10 and the volume of the passageway 35 is reduced as well. While the second device 10 continues to provide a gastric outlet obstruction 19 in the stomach C that blocks the normal passage of chyme from the stomach C and that redirects chyme into the passageway 35, the passageway 35 has reduced dimensions that reduce the volume of chyme that may pass through the device 10. Because less chyme is able to pass through the passageway of the second device 10 compared to the passageway of the first device 10, a greater amount of chyme accumulates within the stomach C leading the patient to feel full and stop consuming food. This will lead to weight loss during the early phase of the second treatment step, however, the patient's digestive tract will adapt to the second device 10 over time. Accordingly, the patient will not feel full after consuming a similar quantity of food, and the weight loss experienced during the early phase will again stagnate.

To combat the stagnation in weight loss experienced during the second step, a third treatment step involves replacing the second device 10 with a different-sized third device 10. Specifically, the second device 10 is replaced by the third device 10 which has an internal passageway 35 with a further reduced diameter D4, such as 2.0 mm. Thus, the passageway diameter D4 of the third device 10 is less than that of both the first and second devices 10. Like the first and second devices 10, the third device 10 provides a gastric outlet obstruction 19 in the stomach C that blocks the normal passage of chyme from the stomach C and that redirects chyme into the passageway 35. Due to the reduced diameter D4, the passageway 35 accepts an even smaller volume of chyme for transmission through the pylorus A and to the duodenum B. Because less chyme is able to pass through the passageway 35 of the third device 10 compared to the passageway 35 of both the first and second devices 10, an even greater amount of chyme accumulates proximate the first bulb 15 and within the stomach C leading the patient to feel full and stop consuming food.

Assuming the same quantity of food is consumed in the same time interval with each of the three different sized devices 10, a greater amount of chyme is transmitted through the passageway 35 of the first device 10 compared to either of the second or third devices 10. Also, a greater amount of chyme is transmitted through the passageway 35 of the second device 10 compared to the third device 10. Under these same conditions, the quantity of chyme obstructed by the bulb 15 and accumulating within the stomach corpus G is greater for the third device 10 compared to the second device 10, and greater for the second device 10 compared to the first device 10. Therefore, a method of treating obesity with the implantation of devices 10 having different sized internal passageways 35 is provided which reduce the volume of chyme passing through the body 30, increase the accumulation of chyme within the stomach C, , and hasten the patient to feel full and reduce or halt food consumption. Because this method of treatment counters the digestive tract's natural tendency to accommodate a single device 10, the patient should continue to experience weight loss.

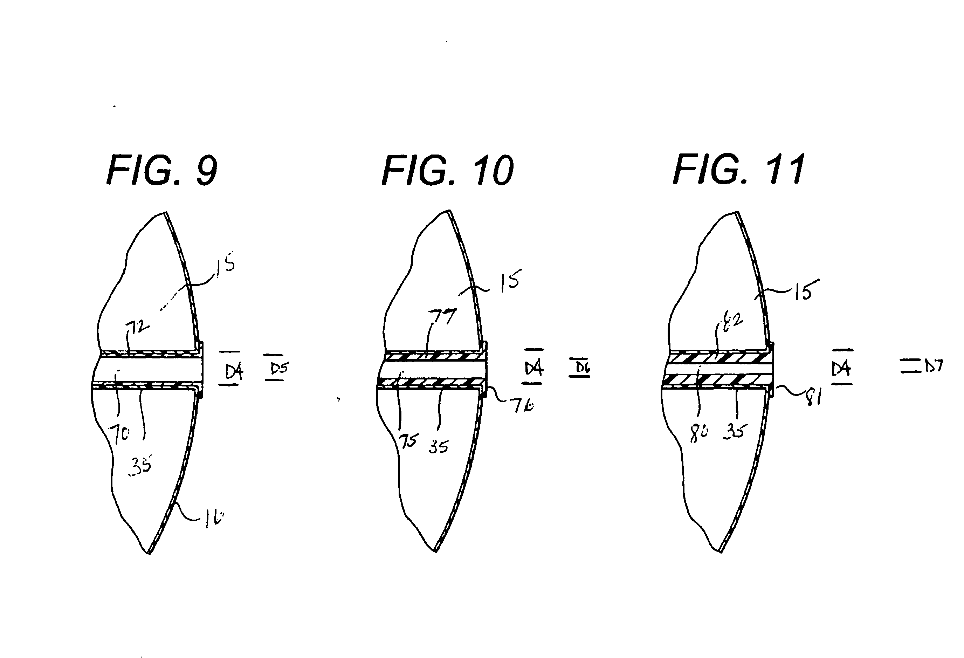

A second obesity treatment method is shown in Figs. 8-11 and involves the use of removable inserts 70 of variable dimensions in the passageway 35 to counter the digest tract's accommodation of the device 10. In a first stage of the treatment method, the device 10 is implanted within the patient's pylorus A. Consistent with that explained above, the first bulb 15 provides a gastric outlet obstruction 19 in the stomach C that blocks the normal passage of chyme from the stomach C and that redirects chyme into the passageway 35 for transmission to the duodenum B. When the patient's digestive tract begins to accommodate the device 10 and weight loss stagnates, the second stage of the treatment method commences. Instead of replacing the existing device 10 with a device 10 having a smaller passageway 10, the second stage involves the insertion of a first insert 70, with the endoscope H, into the passageway 35 of the existing, implanted device 10 (see Figs. 8 and 9). Since the device 10 is already inflated, the passageway 35 slidingly receives the first insert 70. The first insert 70 includes opposed end flanges 71 which engage the outer surfaces 16, 21 of the first and second bulbs 15, 20, respectively, to further secure the position of the insert 70 within the passageway 35. As mentioned above, the passageway 35 has a diameter D4 and the first insert 70 has a diameter D5 that is less than the passageway diameter D4. For example, the passageway diameter D4 is 5 mm and the first insert diameter D5 is 4 mm. The insert 70 has a wall arrangement 72 with a thickness that provides the insert diameter D5. Due to its reduced dimensions, the first insert 70 effectively reduces the volume of the passageway 35 which restricts the amount of chyme that may pass through the device 10 and which increases the accumulation of chyme within the stomach C, leading the patient to feel full and stop consuming food.

A second treatment stage is designed to combat the stagnation in weight loss experienced during the first stage by removing the first insert 70 and inserting a second insert 75 into the passageway 35 with an endoscope H. As shown in Fig. 10, the second insert 75 has a diameter D6 that is less than both the passageway diameter D4 and the first insert diameter D5. For example, the passageway diameter D4 is 5 mm, the first insert diameter D5 is 4 mm, and the second insert diameter D6 is 3 mm. The second insert 75 has an end flange 76 and a wall arrangement 77 with a thickness that provides the insert diameter D6. Like the first insert 70, the second insert 75 effectively reduces the volume of the passageway 35 which thereby restricts the amount of chyme that may pass through the device 10 and into the duodenum B. This restriction increases the accumulation of chyme within the stomach C, leading the patient to feel full and stop consuming food. To offset the stagnation in weight loss that may result in the second stage, a third treatment stage involves using the endoscope H to replace the second insert 75 with a third insert 80 in the passageway 35. Referring to Fig. 11, the third insert 80 has a diameter D7 that is less than the passageway diameter D4 and both the first and second insert diameters D5, D6. As an example, the passageway diameter D4 is 5 mm, the first insert diameter D5 is 4 mm, the second insert diameter D6 is 3 mm, and the third insert diameter D7 is 2 mm. The third insert 80 has an end flange 81 and a wall arrangement 82 with a thickness that provides the insert diameter D7. Like the first and second inserts 70, 75, the third insert 80 effectively reduces the volume of the passageway 35 which thereby restricts the amount of chyme that may pass through the device 10 and into the duodenum B for appetite suppression purposes.

Each of these methods provide sequenced protocols for treating obesity with the device 10. Because the device 10 is easy to implant, either treatment method may be utilized during an office visit without requiring a costly and time-consuming hospital visit for the patient. Once sufficiently inflated, the resulting obstruction causes chyme to accumulate proximate the first bulb 15 or to entering the internal passageway 35 where it is then transported through the inflatable body 35 and discharged from the second bulb 20 into the patient's duodenum B. The accumulation of chyme in the pyloric antrum D and the stomach A causes the patient to feel full and stop eating. Thus, these treatment methods provide a gastric outlet obstruction 19 that slows the passage of chyme into the duodenum B and as a result, the patient feels full and stops eating after consuming relatively small portions. Due to the sequenced nature of the first and second treatment methods, the gastric outlet obstruction 19 counteracts the stomach's attempt to accommodate the device 10 over time.

## Claims

1. A weight control device (10) that is implantable with an endoscope (H) and that is adapted to form a gastric outlet obstruction (19) in a patient's digestive tract, the weight control device (10) comprising:
a first bulb (15), a second bulb (20) with a circumference less than a circumference of the first bulb (15), and an intermediate portion (25) with a circumference less than the circumference of the first and second bulbs (15, 20) that is adapted to reside within the patient's pyloric valve (F) when the device is implanted, wherein the first bulb (15), the second bulb (20) and the intermediate portion (25) collectively define an inflatable body (30);
the body (30) further having at least one internal central passageway (35) extending through the first and second bulbs (15, 20) and the intermediate portion (25); and,
wherein chyme exiting the patient's stomach passes through the internal central passageway to reach the duodenum;
**characterized in that** the weight control device further comprises:
an internal valve assembly (65) having both a valve body (60) and a stem (67), the valve body having one of a one-way valve and a two-way valve, the internal valve assembly (65) extending inward from an off-center position of the outer surface of the body (30) to the first bulb (15), the internal valve assembly (65) providing delivery of an inflation fluid to the first bulb (15);
wherein the first bulb (15), the second bulb (20) and the intermediate portion (25) are formed from one material; and
wherein the intermediate portion (25) is formed with a greater rigidity than the rigidity of the first and second bulbs (15, 20).

2. The weight control device of Claim 1, wherein the first bulb (15) defines an obstruction (19) that is adapted to substantially prevent chyme, other than that passing through the internal central passageway (35), from reaching the pyloric valve (F) and the duodenum (B).

3. The weight control device of Claim 1, wherein the first bulb (15) is adapted to engage an inner surface of the stomach (D) proximate the pyloric valve (F) to prevent the first bulb (15) from moving past the pyloric valve (F) and into the duodenum (B), and wherein the second bulb (20) is adapted to engage an inner surface of the pyloric canal (E) proximate the pyloric valve (F).

4. The weight control device of Claim 3, wherein the intermediate portion (25) is adapted to engage an inner surface of the pyloric valve (F).

5. The weight control device of Claim 1, wherein the internal valve assembly (65) further comprises a severable fill tube.

6. The weight control device of Claim 1, wherein the body (30) has a dumbbell configuration, wherein the transition from the first bulb (15) to the intermediate portion (25) defines a first annular shoulder (18) that engages the terminal portion of the stomach (D) prior to the pyloric valve (F).

7. The weight control device of Claim 1, wherein the internal central passageway (35) is centrally positioned along a longitudinal axis of the body (30) and the valve assembly (65) is offset from the internal central passageway (35).

8. The weight control device of Claim 1, wherein the internal central passageway (35) extends from an end wall (16) of the first bulb (15) through the intermediate portion (25) and to an end wall (21) of the second bulb (20).

9. The weight control device of Claim 8, wherein the internal central passageway (35) includes a first end (35a) aligned with an opening (16a) in the end wall (16) of the first bulb (15) and a second end (35b) aligned with an opening (21a) in the end wall (21) of the second bulb (20).

10. The weight control device of Claim 1, further comprising a first insert (70) that is removably inserted within the internal central passageway (35), the first insert (70) having a diameter (D5) that is less than a diameter (D4) of the internal central passageway (35).

11. The weight control device of Claim 10, wherein the first insert (70) comprises opposed end flanges that engage the outer surface of the first and second bulbs (15, 20).

12. The weight control device of Claim 1, further comprising a second internal passageway (136) extending between the end wall (116) of the first bulb (115) and the end wall (121) of the second bulb (120) and through the intermediate portion (125).

13. The weight control device of Claim 11, wherein the first and second internal passageways (135, 136) converge to form a common exit passageway in the second bulb (120).

## Patentansprüche

1. Gewichtskontrollvorrichtung (10), die mit einem Endoskop (H) implantierbar ist und die geeignet ist, ein Magenausgangshindernis (19) in einem Verdauungstrakt eines Patienten zu bilden, wobei die Gewichtskontrollvorrichtung (10) umfasst:
einen ersten Kolben (15), einen zweiten Kolben (20) mit einem kleineren Umfang als dem Umfang des ersten Kolbens (15) und einem Zwischenabschnitt (25) mit einem kleineren Umfang als dem Umfang der ersten und zweiten Kolben (15, 20), der geeignet ist, sich innerhalb der Pylorusklappe (F) des Patienten zu befinden, wenn die Vorrichtung implantiert ist, wobei der erste Kolben (15), der zweite Kolben (20) und der Zwischenabschnitt (25) gemeinsam einen aufblasbaren Körper (30) definieren;
wobei der Körper (30) ferner wenigstens einen inneren zentralen Durchgang (35) hat, der sich durch die ersten und zweiten Kolben (15, 20) und den Zwischenabschnitt (25) erstreckt; und
wobei Speisebrei, der den Magen des Patienten verlässt, den inneren zentralen Durchgang durchläuft, um den Zwölffingerdarm zu erreichen;
**dadurch gekennzeichnet, dass** die Gewichtssteuervorrichtung ferner aufweist:
eine innere Ventilanordnung (65) sowohl mit einem Ventilkörper (60) als auch einem Schaft (67), wobei der Ventilkörper ein Einwegventil oder ein Zweiwegventil hat, wobei die innere Ventilanordnung (65) sich von einer exzentrischen Position der Außenfläche des Körpers (30) zu dem ersten Kolben (15) einwärts erstreckt, wobei die innere Ventilanordnung (65) die Abgabe eines Aufblasfluids an den ersten Kolben (15) bereitstellt;
wobei der erste Kolben (15), der zweite Kolben (20) und der Zwischenabschnitt (25) aus einem Material ausgebildet sind; und
wobei der Zwischenabschnitt (25) mit einer größeren Steifheit als der Steifheit der ersten und zweiten Kolben (15, 20) ausgebildet ist.

2. Gewichtskontrollvorrichtung nach Anspruch 1, wobei der erste Kolben (15) ein Hindernis (19) definiert, das geeignet ist, um zu verhindern, dass Magenbrei, außer dem, der den inneren zentralen Durchgang (35) durchläuft, die Pylorusklappe (F) und den Zwölffingerdarm (B) erreicht.

3. Gewichtskontrollvorrichtung nach Anspruch 1, wobei der erste Kolben (15) geeignet ist, nahe der Pylorusklappe (F) an einer Innenfläche des Magens (D) anzugreifen, um zu verhindern, dass der erste Kolben (15) sich an der Pylorusklappe (F) vorbei und in den Zwölffingerdarm (B) bewegt, und wobei der zweite Kolben (20) geeignet ist, nahe der Pylorusklappe (F) an einer Innenfläche des Pyloruskanals (E) anzugreifen.

4. Gewichtskontrollvorrichtung nach Anspruch 3, wobei der Zwischenabschnitt (25) an einer Innenfläche der Pylorusklappe (F) angreift.

5. Gewichtskontrollvorrichtung nach Anspruch 1, wobei die innere Ventilanordnung (65) ferner eine trennbare Füllrohrleitung umfasst.

6. Gewichtskontrollvorrichtung nach Anspruch 1, wobei der erste Kolben (15) einen Hantelaufbau hat, wobei der Übergang von dem ersten Kolben (15) zu dem Zwischenabschnitt (25) einen ersten ringförmigen Ansatz (18) definiert, der an dem Abschlussabschnitt des Magens (D) vor der Pylorusklappe (F) angreift.

7. Gewichtskontrollvorrichtung nach Anspruch 1, wobei der innere zentrale Durchgang (35) entlang einer Längsachse des Körpers (30) zentral positioniert ist, und die Ventilanordnung (65) von dem inneren zentralen Durchgang (35) verschoben ist.

8. Gewichtskontrollvorrichtung nach Anspruch 1, wobei der innere zentrale Durchgang (35) sich von einer Endwand (16) des ersten Kolbens (15) durch den Zwischenabschnitt (25) und zu einer Endwand (21) des zweiten Kolbens (20) erstreckt.

9. Gewichtskontrollvorrichtung nach Anspruch 8, wobei der innere zentrale Durchgang (35) ein erstes Ende (35a), das mit einer Öffnung (16a) in der Endwand (18) des ersten Kolbens (15) ausgerichtet ist, und ein zweites Ende (35b), das mit einer Öffnung (21a) in der Endwand (21) des zweiten Kolbens (20) ausgerichtet ist, umfasst.

10. Gewichtskontrollvorrichtung nach Anspruch 1, die ferner einen ersten Einsatz (70) umfasst, der entfernbar in den zentralen Durchgang (35) eingesetzt ist, wobei der erste Einsatz (70) einen Durchmesser (D5) hat, der kleiner als ein Durchmesser (D4) des inneren zentralen Durchgangs (35) ist.

11. Gewichtskontrollvorrichtung nach Anspruch 10, wobei der erste Einsatz (70) entgegengesetzte Endflansche umfasst, die an der Außenfläche der ersten und zweiten Kolben (15, 20) angreifen.

12. Gewichtskontrollvorrichtung nach Anspruch 1, die ferner einen zweiten inneren Durchgang (136) umfasst, der sich zwischen der Endwand (116) des ersten Kolbens (115) und der Endwand (112) des zweiten Kolbens (120) und durch den Zwischenabschnitt (125) erstreckt.

13. Gewichtskontrollvorrichtung nach Anspruch 11, wobei die ersten und zweiten inneren Durchgänge (135, 136) konvergieren, um einen gemeinsamen Ausgangsdurchgang in dem zweiten Kolben (120) zu bilden.

## Revendications

1. Dispositif de contrôle de poids (10) qui est implantable avec un endoscope (H) et qui est adapté à former une obstruction de sortie gastrique (19) dans le tractus digestif d'un patient, le dispositif de contrôle de poids (10) comprenant :
une première ampoule (15), une seconde ampoule (20) avec une circonférence inférieure à une circonférence de la première ampoule (15), et une portion intermédiaire (25) avec une circonférence inférieure à la circonférence des première et seconde ampoules (15, 20) qui est adaptée à résider au sein de la valve pylorique (F) du patient lorsque le dispositif est implanté, dans lequel la première ampoule (15), la seconde ampoule (20) et la portion intermédiaire (25) définissent collectivement un corps gonflable (30) ;
le corps (30) ayant en outre au moins un passage central interne (35) s'étendant à travers les première et seconde ampoules (15, 20) et la portion intermédiaire (25) ; et
dans lequel le chyme sortant de l'estomac du patient passe à travers le passage central interne pour atteindre le duodénum ;
**caractérisé en ce que** le dispositif de contrôle de poids comprend en outre :
un ensemble de valve interne (65) ayant à la fois un corps de valve (60) et une tige (67), le corps de valve ayant une d'une valve unidirectionnelle et d'une valve bidirectionnelle, l'ensemble de valve interne (65) s'étendant vers l'intérieur depuis une position excentrée de la surface externe du corps (30) vers la première ampoule (15), l'ensemble de valve interne (65) fournissant la livraison d'un fluide de gonflage à la première ampoule (15) ;
dans lequel la première ampoule (15), la seconde ampoule (20) et la portion intermédiaire (25) sont formées d'un matériau ; et
dans lequel la portion intermédiaire (25) est formée avec une rigidité supérieure à la rigidité des première et seconde ampoules (15, 20).

2. Dispositif de contrôle de poids selon la revendication 1, dans lequel la première ampoule (15) définit une obstruction (19) qui est adaptée à essentiellement empêcher au chyme, autre que de passer à travers le passage central interne (25), d'atteindre la valve pylorique (F) et le duodénum (B).

3. Dispositif de contrôle de poids selon la revendication 1, dans lequel la première ampoule (15) est adaptée à s'engager avec une surface interne de l'estomac (D) à proximité de la valve pylorique (F) pour empêcher la première ampoule (15) de se déplacer au-delà de la valve pylorique (F) et dans le duodénum (B), et dans lequel la seconde ampoule (20) est adaptée à s'engager avec une surface interne du canal pylorique (E) à proximité de la valve pylorique (F).

4. Dispositif de contrôle de poids selon la revendication 3, dans lequel la portion intermédiaire (25) est adaptée à s'engager avec une surface interne de la valve pylorique (F).

5. Dispositif de contrôle de poids selon la revendication 1, dans lequel l'ensemble de valve interne (65) comprend en outre un tube de remplissage divisible.

6. Dispositif de contrôle de poids selon la revendication 1, dans lequel le corps (30) a une configuration en forme d'haltère, dans lequel la transition de la première ampoule (15) à la portion intermédiaire (25) définit un premier épaulement annulaire (18) qui s'engage avec la portion terminale de l'estomac (D) avant la valve pylorique (F).

7. Dispositif de contrôle de poids selon la revendication 1, dans lequel le passage central interne (35) est positionné centralement le long d'un axe longitudinal du corps (30) et l'ensemble de valve (65) est décalé du passage central interne (35).

8. Dispositif de contrôle de poids selon la revendication 1, dans lequel le passage central interne (35) s'étend depuis une paroi d'extrémité (16) de la première ampoule (15) à travers la portion intermédiaire (25) et vers une paroi d'extrémité (21) de la seconde ampoule (20).

9. Dispositif de contrôle de poids selon la revendication 8, dans lequel le passage central interne (35) inclut une première extrémité (35a) alignée avec une ouverture (16a) dans la paroi d'extrémité (16) de la première ampoule (15) et une seconde extrémité (35b) alignée avec une ouverture (21a) dans la paroi d'extrémité (21) de la seconde ampoule (20).

10. Dispositif de contrôle de poids selon la revendication 1, comprenant en outre un premier insert (70) qui est inséré de manière amovible au sein du passage central interne (35), le premier insert (70) ayant un diamètre (D5) qui est inférieur à un diamètre (D4) du passage central interne (35).

11. Dispositif de contrôle de poids selon la revendication 10, dans lequel le premier insert (70) comprend des brides d'extrémité opposées qui s'engagent avec la surface externe des première et seconde ampoules (15, 20).

12. Dispositif de contrôle de poids selon la revendication 1, comprenant en outre un second passage interne (136) s'étendant entre la paroi d'extrémité (116) de la première ampoule (115) et la paroi d'extrémité (121) de la seconde ampoule (120) et à travers la portion intermédiaire (125).

13. Dispositif de contrôle de poids selon la revendication 11, dans lequel les premier et second passages internes (135, 136) convergent pour former un passage de sortie commun dans la seconde ampoule (120).
